# EUROPEAN PATENT SPECIFICATION

(11) **EP 2 345 448 B1**
(45) Date of publication and mention of the grant of the patent: **25.07.2018**
(21) Application number: 11150286.0
(22) Date of filing: 06.01.2011
(51) Int. Cl.: A61M 16/08

(54) **Ventilator device or anaesthetic device**
Beatmungsgerät oder Anästhesiegerät
Appareil de respiration ou appareil d'anesthésie

(30) Priority: 14.01.2010 DE 102010004527
(43) Date of publication of application: 20.07.2011
(73) Proprietor: Drägerwerk AG & Co. KGaA, 23558 Lübeck (DE)
(72) Inventor: Kholtchanski, Grigory, 23566 Lübeck (DE); Lischinski, Robert, 23911 Einhaus (DE); Pasdzior, Sven, 23570 Lübeck-Travemünde (DE); Radomski, Klaus, 23566 Lübeck (DE); Reichert, Dirk-Stefan, 23554 Lübeck (DE)
(74) Representative: Haseltine Lake LLP

(56) References cited:
- WO-A1-2007/051230
- WO-A2-2008/058506
- FR-A1- 2 889 072
- US-A- 5 106 129
- US-A1- 2004 182 392
- US-A1- 2007 163 600

## Description

The present invention relates to a ventilator or anaesthetic machine which comprises at least two modules, such for example as a turbine, a breathing system, a mixer and/or an anaesthetic vaporiser, and at least one pneumatic connecting means connecting two modules pneumatically. Ventilators and anaesthetic machines are used in the field of medical technology for the artificial ventilation of patients and to anaesthetise them. The ventilators and anaesthetic machines comprise in this case a plurality of modules which are connected together pneumatically in a gas-tight fashion by means of a pneumatic connecting means. The modules are for example a turbine, a mixer, an anaesthetic vaporiser or a breathing system. Connected to the breathing system is a breathing hose for maintaining a patient's respiration. At the time of manufacture, the individual modules of the ventilator or anaesthetic machine are first produced separately from and independently of one another and then have to be connected together by means of the pneumatic connecting means. What is generally done in this case is that each pair of modules are connected by a plurality of pneumatic connecting means because a plurality of pneumatic connections are usually required between the pair of modules to enable the ventilator or anaesthetic machine to operate.
What are generally used as connecting means are hoses or, in certain applications, even connecting pipes.
Connecting pipes of this kind are usually connected to the modules with a seal by means of O-rings.
The large number of connections required in a ventilator or anaesthetic machine and hence the large number of hoses and/or connecting pipes result in the assembly operations to connect the modules together being costly and complicated.
The connecting pipes have to be fitted to the modules in precise alignment to ensure that a sealed connection is made indirectly via the O-rings. This being the case, only small tolerances on the vertical, horizontal and axial positions of the modules relative to one another, and of the modules relative to the associated connecting pipes, are permitted at the time of assembly.
The modules having been connected to the hoses and/or connecting pipes, a complicated test has to be carried out to find any leaks, or any hoses which have been mis-inserted relative to one another, because these constitute a major potential risk to patients.
The hoses, plus the appropriate connectors at the ends, have to be of a certain minimum length as a function of their diameter, and as a function of the radiuses to which they can be bent, to enable the hoses to be inserted in the connections at the modules.
When the distance between two modules is short, the only possible way of doing this is with a large loop, which means that a hose of considerable length, with a correspondingly high pneumatic resistance and volume of dead space, is required. What is also required is a relatively large minimum distance between the modules to be connected.
US 3,394,954 discloses a coupling device for medical apparatus, for connecting two flexible tubes. This means that a flexible tube is necessary, which is a disadvantage.
US 4,870,961 discloses a pneumatic connecting means for connecting a breathing hose to a medical ventilator.
The object of the present invention is therefore to provide a ventilator or anaesthetic machine in which the modules of the ventilator or anaesthetic machine can be easily and reliably connected together pneumatically while only a small amount of space is required.

This object is achieved by ventilator or anaesthetic as claimed in claim 1. Optional features of the present invention are recited in the dependent claims. The modules of a ventilator or anaesthetic machine can thus easily be connected together by the connecting tube without a flexible hose. A secure and reliable connection can be made between two modules in this way, a reliable pneumatic connection being able to be obtained with little in the way of costly or complicated assembly work. Also the distance between the two modules required for making the pneumatic connection is only small, thus enabling a saving to be made on the space taken up by the ventilator or anaesthetic machine. What is more, no loops are required as would be the case with a flexible hose connection, which reduces pneumatic resistances and volumes of dead space in the gas-carrying system of the ventilator or anaesthetic machine. Also, mistakes in plugging hose connections together are avoided.
The connecting tube is, in particular, a separate component and/or is not a bendable hose. The connecting tube is a separate component, i.e. the connecting tube is not connected to at least one module by a physical union. The connecting tube thus constitutes a separate component which is produced separately from and independently of the module.
In a further embodiment, the connecting tube is connected to the first module and/or the second module by a frictional and/or positively interengaging connection.
In the case of a connecting tube which is circular in cross-section, the recess takes the form in this case of, for example, a bore or a milled aperture.
The connecting tube preferably comprises an inner material on the inside and an outer material on the outside. The connecting tube thus has an inner tube composed of the inner material and an outer tube composed of the outer material. The outer tube is preferably fastened to the inner tube by a physical union in this case, such as by vulcanisation for example. As well as this, the outer tube may also be fastened to the inner tube by adhesive bonding for example.

In a variant, the inner material has a higher modulus of elasticity than the outer material. The modulus of elasticity of the inner material is usefully 2, 5, 10, 30 or 50 times higher than the modulus of elasticity of the outer material.

The outer material is preferably formed by an elastic substance, such for example as an elastomer which is resistant to corrosive media such as anaesthetic gases (nitrous oxide, oxygen) and volatile anaesthetics such as halothane, enflurane, desflurane, isoflurane or sevoflurane. Suitable elastic materials which are resistant to the said media are for example materials from the group comprising ethylene propylene rubber compounds and ethylene propylene elastomers (EPDM) which are known for example by the trademarks Vistalon®, Dutral®, Buna AP® or APTK®, fluoro rubber compounds (FKM, FPM) which are known by the trademarks Viton®, Tecnoflon® or Fluorel®, and perfluoro rubber compounds (FFKM, FFPM) which are known by the trademarks Parafluor® or Kalrez®.

In a further embodiment, the inner material is a corrosion-resistant metal and in particular stainless steel. The gas of the ventilator or anaesthetic machine flows through the inner tube. It makes good sense for the inner tube to be made from stainless steel because it is possible by this means to provide a flow duct to carry the gas which is corrosion-resistant, durable and resistant to aggressive media and in particular to volatile anaesthetics such as halothane, isoflurane, desflurane, sevoflurane or enflurane.

In a similar and equally good form to an embodiment where the inner material is stainless steel, an embodiment where the inner material is polyether ether ketone (PEEK) ensures resistance to corrosive and aggressive media of the kind represented by, for example, volatile anaesthetics such as halothane, isoflurane, desflurane, sevoflurane or enflurane.

The sealing bead preferably takes the form of a complete surrounding loop.

Also as a preference, the sealing bead is made of the same elastic material as the outer material and, as a further preference, takes the form of ethylene propylene rubber (EPDM), fluoro rubber (FKM, FPM) or perfluoro rubber (FFKM, FFPM).

In a supplementary variant, the connecting tube is circular in cross-section.

In a further variant, the cross-sectional shape of the recess in the first and/or second module corresponds to the external cross-sectional shape of the connecting tube at the first end, and in particular at the sealing bead.

The external shape of the connecting tube at the first and/or second end and the shape of the recess are of substantially identical forms to enable a complete sealing area to be formed which extends round in a loop.

What in particular ensures that both a horizontal and a vertical and even an angular offset between the modules can be compensated for is a circular form for the connecting tube and the recesses in the modules.

In a further embodiment, the cross-sectional shape of the recess in the first and/or second module corresponds to the external cross-sectional shape of the connecting tube at the first and/or second end and in particular at the sealing bead. In the state where it is not inserted in the recess in the first module, the first and/or second end of the connecting tube, and in particular its sealing bead, is formed in this case to be slightly larger than the recess in the first module, thus causing the first and/or second end of the connecting tube to be arranged in the recess in the first and/or second module under a pre-loading. The first and/or second end of the connecting tube, and in particular the sealing bead, are thus arranged in the recess in the first and/or second module under an elastic pre-loading. The pre-loading is produced by elastic deformation of the elastic sealing bead. A compressive stress thus occurs, at the sealing area, between the first and/or second end of the connecting tube and the boundary of the recess which is formed by the first and/or second module, thus ensuring that there is a secure and reliable seal, at the recess, between the first and/or second end of the connecting tube and the first and/or second module. Because of the elastic deformability mentioned, it is also possible for inaccuracies in production to be compensated for in an advantageous way at the ends of the connecting tubes and at the recesses in the modules.

In a further embodiment, the sealing bead is formed to be co-axial with an axis of the first and/or second connecting tube.

In a further embodiment, the first and/or second connecting tube is co-axially arranged in the recess in the first and/or second module.

In what follows, embodiments of the invention are described in detail, by way of example only, by reference to the accompanying drawings of which:
Fig. 1 is a highly simplified longitudinal section through a ventilator having two modules,
Fig. 2 is a highly simplified longitudinal section through an anaesthetic machine having four modules,
Fig. 3 is a longitudinal section through a first embodiment of connecting tube in a recess in a second module,
Fig. 4 is a longitudinal section through a second embodiment of connecting tube in the recess in the second module,
Fig. 5 is a longitudinal section through a third embodiment of connecting tube in the recess in the second module,
Fig. 6a is a detailed longitudinal section, showing dimensions, through a connecting tube and two modules in a first possible assembly,
Fig. 6b is a longitudinal section through two connecting tubes and a first and second module in a possible first assembly,
Fig. 7 is a longitudinal section through two connecting tubes and the first and second modules in a possible second assembly,
Fig. 8 is a longitudinal section through two connecting tubes and the first and second modules in a possible third assembly,
Fig. 9 is a longitudinal section through two connecting tubes and the first and second modules in a possible fourth assembly,
Fig. 10 is a longitudinal section through two connecting tubes and the first and second modules in a possible fifth assembly.

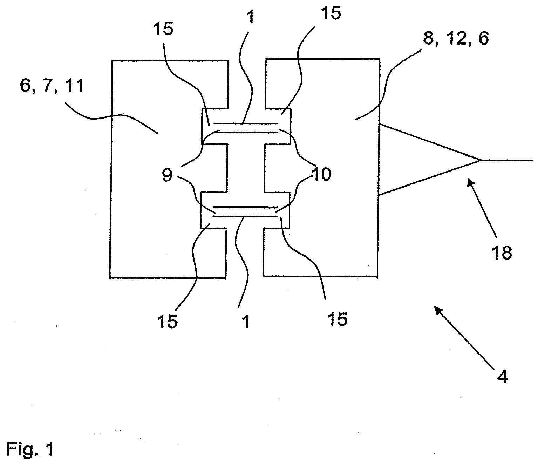

A ventilator 4 is shown in a highly simplified form in Fig. 1. The ventilator 4 comprises two modules 6, namely a first module 7 and a second module 8. The two modules 6 are connected together pneumatically in this case by two connecting tubes 1. Each module 6 has two recesses 15 in this case in which respective ends 9, 10 of the connecting tube 1 are arranged. The first module 7 represents a turbine 11 in this case and the second module 8 represents a breathing system 12 in this case. Connected to the breathing system 12 is a breathing hose 18.

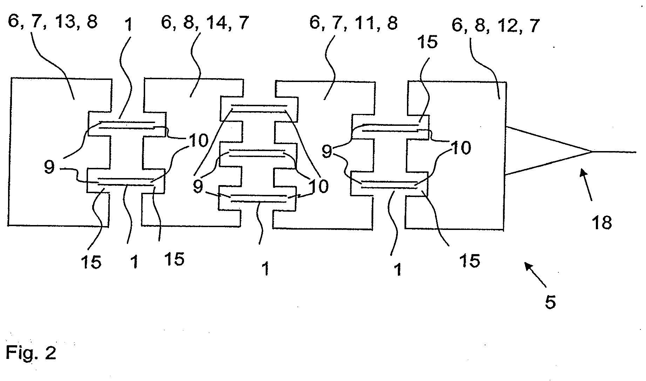

In a similar way to Fig. 1, Fig. 2 shows an anaesthetic machine 5. The anaesthetic machine 5 comprises in this case four modules 6 which, in a similar way to what is done in the first embodiment shown in Fig. 1, are connected together pneumatically by means of connecting tubes 1. The connecting tubes 1 are arranged at each of their ends 9, 10 in recesses 15 in the four modules 6 of the anaesthetic machine 5 in this case. As well as the turbine 11 and breathing system 12, the anaesthetic machine 5 comprises in this case a mixer 13 and an anaesthetic vaporiser 14. The anaesthetic vaporiser 14 can be connected to an anaesthetic metering unit by a connecting line (not shown). Connected to the breathing system 12 is the breathing hose 18 for maintaining the patient's respiration artificially.

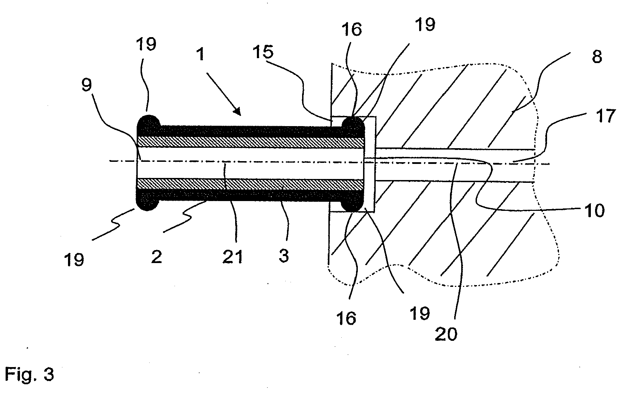

Shown in Figs. 3 to 5 are three different embodiments of connecting tube 1. Only the second module 8 is shown in Figs. 3 to 5 in this case. The first module 7 is not shown in Figs. 3 to 5. Also shown is a line of symmetry 20 about which the air duct 17 in the second module 8 is axially symmetrical and a line of symmetry 21 about which the connecting tube 1 is axially symmetrical. Where the air duct 17 in the second module 8 is aligned in the way shown in a straight line with the connecting tube 1, the lines of symmetry 20, 21 become a single line of symmetry which is identical in space.

In the first embodiment of connecting tube 1 which is shown in Fig. 3, the connecting tube 1 has an outer tube 2 of an elastic outer material and an inner tube 3 of a non-elastic inner material. The outer tube 2 is fastened in this case to the inner tube 3 by a physical union by being vulcanised thereto. The connecting tube 1 has a first end 9 and a second end 10. The second end 10 of the connecting tube 1 is arranged in this case in the recess 15 in the second module 8. Formed at the first and seconds ends 9, 10 of the connecting tube 1 in this case is a sealing bead 19 of the outer material, namely the elastomer, which extends round in a loop. The connecting tube 1 is circular in cross-section and the recess 15 constitutes a bore which is circular in cross-section. The external cross-sectional shape of the second end 10 of the connecting tube 1, namely in particular the sealing bead 19 at the second end 10, thus corresponds to the cross-sectional shape of the recess 15 which is circular in cross-section. When the connecting tube 1 is introduced into the recess 15, a sealing area 16 extending round in a complete loop can thus be produced between the sealing bead 19 and the second module 8 at the recess 15.

In a state where the connecting tube 1 is not inserted in the recess 15, the sealing bead 19 is slightly larger than the recess 15 and, due to its elastic properties, the sealing bead 19 is thus deformed elastically when it is inserted in the recess 15, i.e. the circumference of the sealing bead 19 is made smaller. The sealing bead 19 is thus arranged in the recess 15 under a pre-loading and a secure and reliable seal is thus ensured between the sealing bead 19 and the second module 8 at the recess 15 due to the compressive forces which come into play. Inaccuracies in production at the connecting tube 1 and the recess 15 can also be compensated for in this way. The first end 9 of the connecting tube 1 is also arranged, in a similar way to the second end 10, in a recess 15 in the first module 7, although this is not shown. From the second module 8 for example, the air is thus able to flow through an air duct 17 in the second module 8, then through the connecting tube 1, i.e. through the space connected by the inner tube 3, and then into an air duct 17 in the first module 7 (not shown), as shown in Fig. 3. Due to its geometry and its arrangement in the recess 15, the sealing bead 19 in the first embodiment shown in Fig. 3 makes it possible for the connecting tube 1 to be sealed off radially from the second module 8 at the recess 15.

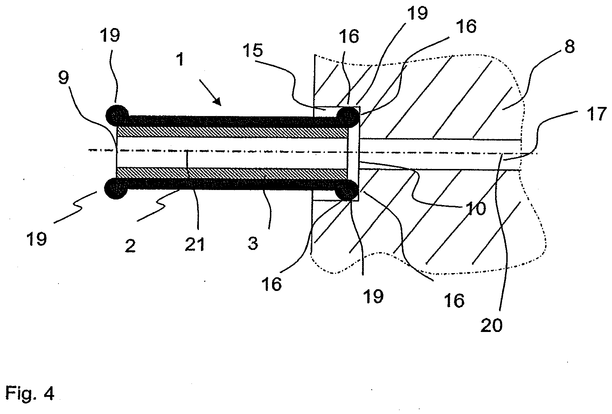

The second embodiment of connecting tube 1 shown in Fig. 4 differs from the first embodiment thereof merely in the geometry of the sealing bead 19. In this case the sealing bead 19 is made longer outwards in the axial direction than the inner tube 3, thus enabling not only a radial sealing area 16 but also an axial sealing area 16 to be produced by means of the sealing bead 19. Otherwise, the second embodiment corresponds to the first embodiment shown in Fig. 3. Elements in the second embodiment in Fig. 4 which are the same as in the first embodiment shown in Fig. 1 have been given the same reference numerals as in Fig. 1.

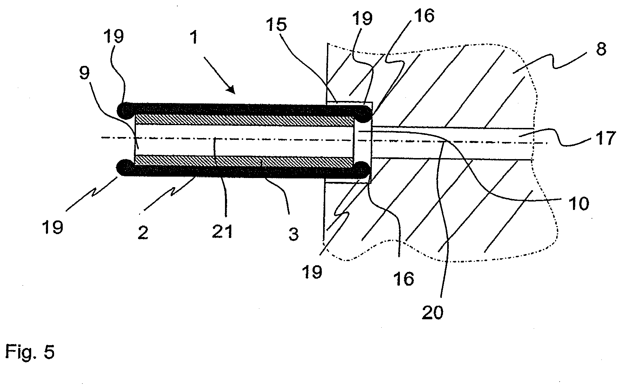

The third embodiment shown in Fig. 5 once again differs from the first embodiment shown in Fig. 3 merely in the geometry of the sealing bead 19. The sealing bead 19 is made longer in the axial direction than the inner tube 3 and is offset inwards radially relative thereto, thus enabling only an axial sealing area 16 to be produced in the recess 15 by means of the sealing bead 19. Otherwise, the third embodiment of connecting tube 1 shown in Fig. 5 corresponds to the first embodiment thereof shown in Fig. 3. Elements in the third embodiment in Fig. 5 which are the same as in the first embodiment shown in Fig. 1 have been given the same reference numerals as in Fig. 1.

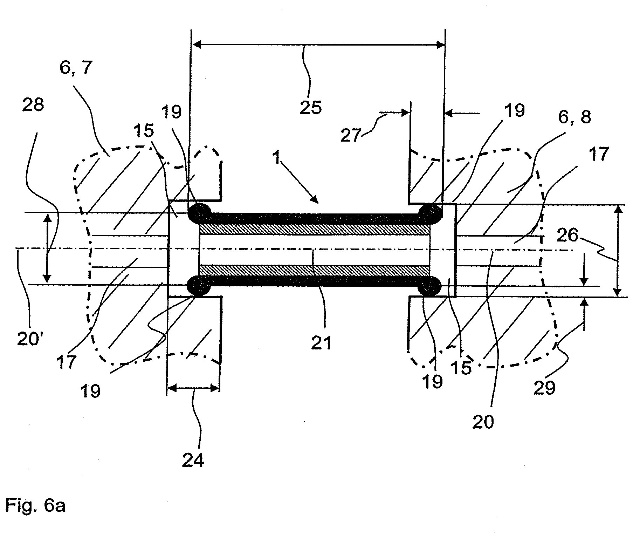

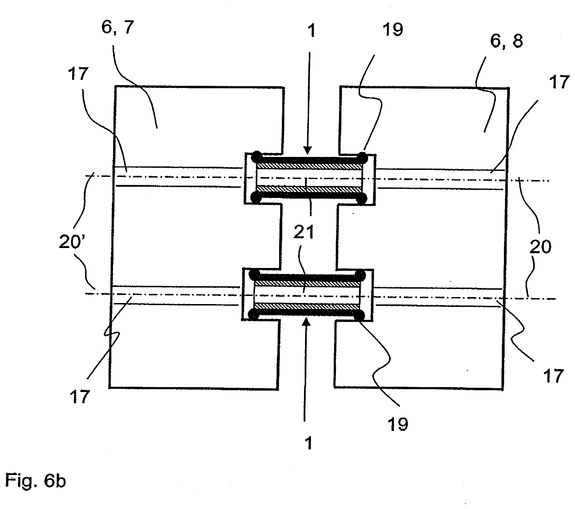

Shown in Figs. 6 to 10 are five different situations for the assembly of a first and second module 7, 8 using in each case two connecting tubes 1. The connecting tubes 1, having sealing beads 19, are arranged in the recesses 15 in the modules 6, 7, 8 and connect the air ducts 17 in the modules 6, 7, 8 together. Shown in Figs. 6 to 10 are lines of symmetry 20, 20', 21 about which the air ducts 17 in the modules 6, 7, 8 and the connecting tube 1 are respectively axially symmetrical, to make clearer, in detail, the positions of the modules 6, 7, 8 relative to one another and the positions of the modules 6, 7, 8 relative to the connecting tubes 1.

To allow things to be shown in detail, only a single connecting tube 1 is shown in the arrangement shown in Fig. 6a, with the arrangement comprising the first module 7 and the second module 8 connected in a straight line with no vertical or angular offset in the positioning of the modules 7, 8 relative to one another. The sealing beads 19 form a seal in the recesses 15 in such a way that there is a gas-tight connection between the air ducts 17 in the first and second modules 7, 8. What are shown by way of example in Fig. 6a, in a representation which is not to scale, are the situations in space of a connecting tube 1, module 6, sealing bead 19, air duct 17 and recess 15.

In the vertical position, the connecting tube 1 is fitted into the recess 15, of a height 26, in each of the modules 6 at its maximum outside diameter comprising the diameter 28 of the connecting tube 1 and the thickness 29 of the sealing bead 19. Along its length 25, the connecting tube 1 is fitted into recesses of a depth 24 to a depth of insertion 27 with the connecting tube 1 in a horizontal position in the recess 15. To illustrate a connection of the modules 6 by means of a connecting tube 1, the length 25 of the pipe may be sized at 35 mm, the depth 26 of the recesses may be sized at 11 mm and the depth of insertion 27 may be at 5 mm in such a way that the connecting tube 1 fits into the recess 15 in a central position horizontally. The height 24 of the recess may be sized at 14 mm such that the connecting tube 1 fits into the recess 15 with a seal if the diameter 28 of the pipe is 10 mm and the thickness of the sealing bead is 2.1 to 2.4 mm.

In the view shown in Fig. 6b, there is no tolerance between the first module 7 and the second module 8, i.e. there is no vertical or angular offset in their geometrical arrangement relative to one another. A connection made by two connecting tubes 1 is shown. There is a secure and reliable pneumatic connection between the first module 7 and the second module 8.

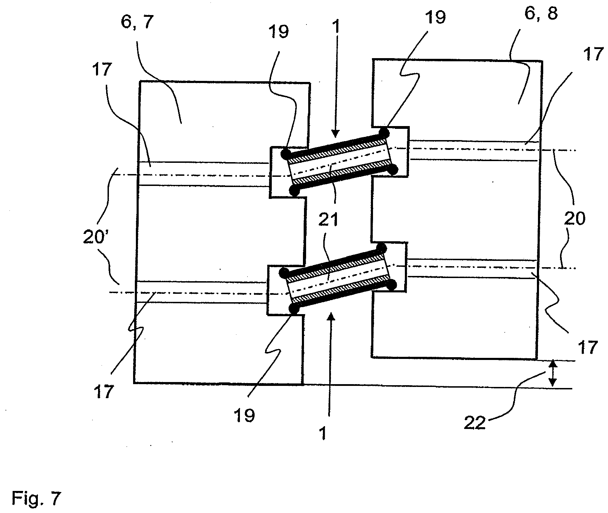

In the possible assembly shown in Fig. 7, the second module 8 is arranged at a slightly higher level than the first module 7, i.e. there is thus a vertical offset 22 between the second module 8 and the first module 7. The connection shown is one made with two connecting tubes 1. Due to the elastic nature of the sealing bead 19, this possible assembly too makes a secure and reliable pneumatic connection possible between the first module 7 and the second module 8.

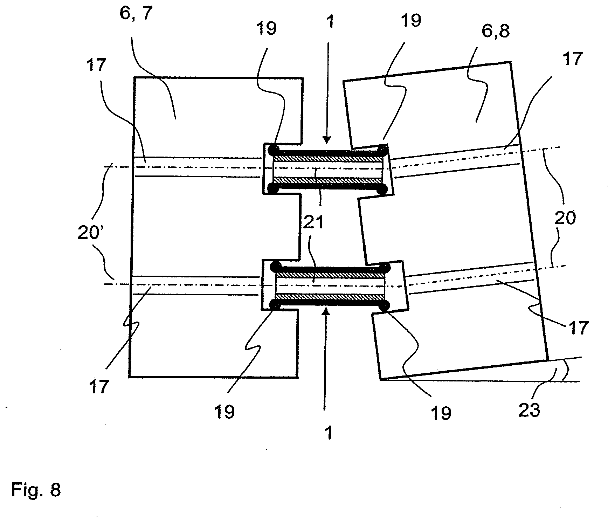

In Fig. 8 there is an angular offset 23 between the first module 7 and the second module 8. What this means is that the distance between the first and second modules 7, 8 is less at the top than it is between the first and second modules 7, 8 at the bottom. The connection shown is one made with two connecting tubes 1. With this situation for installation or possible assembly too, there is an assurance of a reliable pneumatic connection between the first and second modules 7, 8.

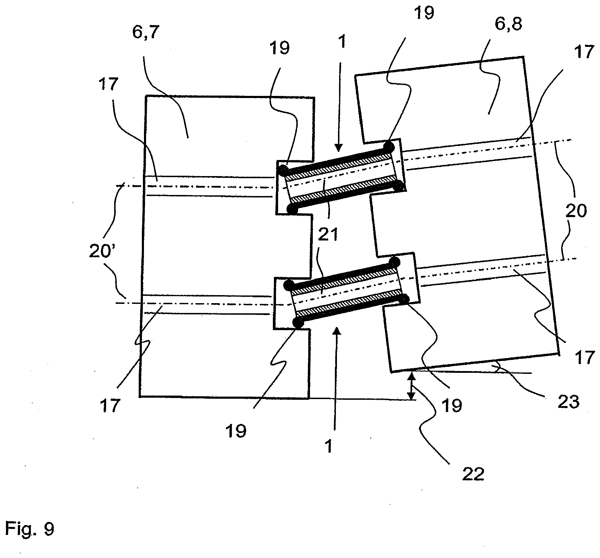

In a fourth possible assembly which is shown in Fig. 9, there is both a vertical offset 22 and an angular offset 23 between the first and second modules 7, 8. The connection shown is one made with two connecting tubes 1. The distance between the first and second modules 7, 8 is smaller at the top than at the bottom in this case and the second module 8 is also arranged at a slightly higher level than the first module 7. With this possible assembly too, there is a reliable pneumatic connection between the first and second modules 7, 8.

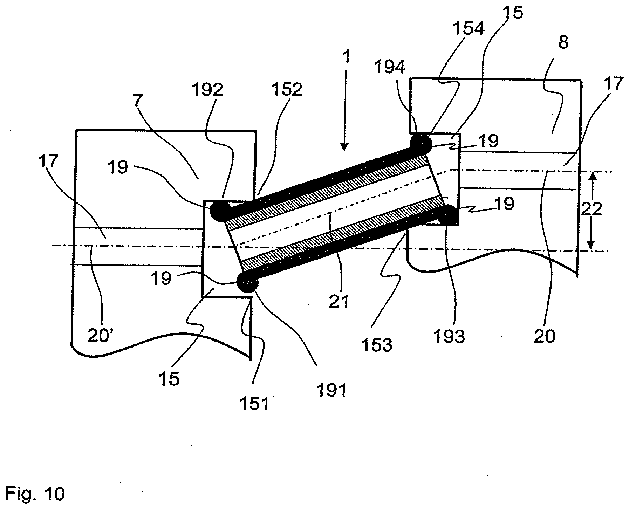

In the fifth possible assembly shown in Fig. 10, a version in which there is an excessive vertical offset 22 is shown to illustrate the limits set by the structure for connecting a first module 7 to a second module 8. The distance between the first and second modules 7, 8 is smaller at the top than at the bottom in this case. At the same time, the second module 8 is arranged is arranged in this case at a more than just slightly higher level than the first module 7. To emphasise the details of the arrangement, a connection made with only one connecting tube 1 is shown in which the arrangement is also being asked to do too much structurally, i.e. the vertical offset 22 can no longer be compensated for. Points of contact 151, 152, 153, 154 in the recesses 15 and points of contact 191, 192, 193, 194 by the sealing beads 19 are shown and it can be seen from the positions of these relative to one another whether a sealed connection is possible. It can be seen that, at least at the point of contact 153 in the recess 15 in the second module 8, the connecting tube 1 is in a tipped position and has an overlap with the second module and that the connecting tube 1 can no longer be fitted into the recess 15 without the connecting tube and/or the second module 8 being deformed mechanically. The result of this is that the connecting tube 1 can no longer make contact with a seal in the recess 19 in the first module 7, at least at the point of contact 191 by the sealing bead 19. The plug-in connection as a whole is thus no longer gas-tight. With a connection of this particular layout, a reliable pneumatic connection between the first and second modules 7, 8 is thus not made by the connecting tube 1 which is used, because the geometry of the connecting tube 1 is not adapted to the positions of the modules 6, 7, 8 and the distance between them.

The first, second, third and fourth possible assemblies or situations for installation show that a secure and reliable pneumatic connection can be made between the first and second modules 7, 8 with the help of the connecting tubes 1 when there are slight differences in the dimensional accuracy of the components or when there is a difference in their structural symmetry and in particular when there is a vertical offset 22 and/or an angular offset 23 relative to one another between the first and second modules 7, 8.

The fifth possible assembly shows the limits set by the structure at which a reliable pneumatic connection cannot be made between the first and second modules 7, 8.

A secure and reliable pneumatic connection between two modules 6 of a ventilator or anaesthetic machine 4, 5 is provided by the connecting tube 1. Appropriate recesses 15 merely have to be formed in the modules 6 of the ventilator or anaesthetic machine 4, 5 at the time of production. The connecting tube 1 simply has to be inserted in these recesses 15. The pneumatic connection can thus easily be made by a plug-in action and can also be disconnected very easily, which is an advantage particularly when maintenance work is being done on the ventilator or anaesthetic machine 4, 5. Hence only a very few components are required for the pneumatic connection, which is an advantage, and costs can thus be reduced on the one hand and reliability at the time of assembly can thus be increased on the other hand.

The length 25 of the connecting tube 1 ranges from 25 mm to 35 mm for example and its outside diameter 28 is approximately 8 mm to 12 mm for example and its outside diameter 28 is preferably 10 mm. As a departure from this, the length 25 of the connecting tube 1 may range from 10 mm to 40 mm for example and its diameter may range from 5 mm to 15 mm for example. The length 25 will be governed essentially by the intended structural distance between the modules 6. The thickness 29 of the sealing bead will be governed by the elasticity and deformability of the elastic material of the sealing bead and will range from 1 mm to 5 mm. If a fluoro rubber is selected as the elastic outer material and the elastic material of the sealing bead, then a thickness of approximately 2 mm for example is advantageous for the sealing bead.

The height 26 and depth 24 of the recess 15 are selected in such a way that there is, within wide limits in the given situations for assembly and with the interaction between the length 25 of the tube, the material of the sealing beads and the thickness 29 of the sealing beads, a secure, gas-tight, pneumatic connection.

The geometry or size of the connecting tube 1 relative to the recess 15 may also be designed in this case in such a way that there is no risk at the time of assembly of a mistake or of mis-insertion in unintended recesses 15 in a module 6.

Seen overall, there are considerable advantages to the ventilator or anaesthetic machine 4, 5 according to the invention. On the one hand, the pneumatic connection between the modules 6 of the ventilator or anaesthetic machine 4, 5 is substantially simplified by the use of a smaller number of components, thus enabling costs to be reduced in production and the possibility of errors in assembly to be substantially ruled out. Also, a highly reliable pneumatic connection is provided which, what is more, also makes possible a short distance between the modules 6, thus advantageously reducing the space required by the ventilator or anaesthetic machine 4, 5.

## Claims

1. A ventilator or anaesthetic machine (4, 5) which comprises at least two modules (6, 7, 8), at least one pneumatic connecting means connecting the two modules (6, 7, 8) pneumatically, and wherein :
the at least one pneumatic connecting means comprises a connecting tube (1) which is surrounded by the first module (7) at a first end (9) and by the second module (8) at a second end (10), a pneumatic connection thus being made between the first module (7) and the second module (8) by the connecting tube (1);
the first and second module (7, 8) each have a recess (15), and the first end (9) of the connecting tube (1) is arranged in the recess (15) in the first module (7) and the second end (10) of the connecting tube (1) is arranged in the recess (15) in the second module (8);
a first elastic sealing bead (19) is formed at the first end (9) of the connecting tube (1) and a second elastic sealing bead (19) is formed at the second end (10) of the connecting tube (1), and the first elastic sealing bead (19) is in contact with the first module (7) and the second elastic sealing bead (19) is in contact with the second module (8), a first sealing area (16) thus being formed between the first elastic sealing bead (19) and the first module (8) and a second sealing area (16) thus being formed between the second elastic sealing bead (19) and the second module (8).

2. The ventilator or anaesthetic machine according to claim 1, in which the connecting tube (1) is a separate component.

3. The ventilator anaesthetic machine according to claim 1 or 2, in which the connecting tube (1) is not a bendable hose.

4. The ventilator or anaesthetic machine according to any of claims 1 to 3, in which the connecting tube (1) is connected to the first module (7) by a frictional or positively interengaging connection.

5. The ventilator or anaesthetic machine according to any of claims 1 to 4, in which the connecting tube (1) is connected to the second module (8) by frictional or positively interengaging connection.

6. The ventilator or anaesthetic machine according to any one of the preceding claims, in which the connecting tube (1) comprises a corrosion-resistant metal on the inside, and an elastic outer material on the outside.

7. The ventilator or anaesthetic machine according to claim 6, in which the corrosion-resistant metal is stainless steel.

8. The ventilator or anaesthetic machine according to any of claims 1 to 5, in which the connecting tube (1) comprises a plastics material on the inside and an elastic outer material on the outside, and in which the plastics material is a polyether ether ketone .

9. The ventilator or anaesthetic machine according to any of claims 6 to 8, in which the elastic outer material is a fluoro rubber, a perfluoro rubber or an ethylene propylene rubber.

10. The ventilator or anaesthetic machine according to any one of claim 6 to 9, and in which the sealing bead (19) is formed using the outer material.

11. The ventilator or anaesthetic machine according to any one of the preceding claims, in which the connecting tube (1) is circular in cross-section.

12. The ventilator or anaesthetic machine according to any preceding claim, in which the cross-sectional shape of the recess (15) in the first module (7) and/or the second module (8) corresponds to the external cross-sectional shape of the connecting tube (1) at the first and/or second end of the connecting tube (1), respectively.

13. The ventilator or anaesthetic machine according to any preceding claim, in which, the cross-sectional shape of the recess (15) in the first module (7) corresponds to the external cross-sectional shape the sealing bead (19).

14. The ventilator or anaesthetic machine according to any preceding claim, in which, in a state where it is not inserted in the recess (15) in the modules (6, 7, 8), the first end (9) and/or second end (10) of the connecting tube (1), and its sealing bead (19), is formed to be slightly larger than the recess (15) in the modules (6, 7, 8), thus causing the first end (9) of the connecting tube (1) to be arranged in the recess (15) in the modules (6, 7, 8) under a pre-loading.

15. The ventilator or anaesthetic machine according to any preceding claim including at least one module selected from the group consisting of a turbine, a breathing system, a mixer and an anaesthetic vaporiser.

## Patentansprüche

1. Beatmungs- oder Anästhesiegerät (4, 5), das wenigstens zwei Module (6, 7, 8), wenigstens ein pneumatisches Verbindungsmittel zur pneumatischen Verbindung der beiden Module (6, 7, 8) umfasst, und wobei:
das wenigstens eine pneumatische Verbindungsmittel ein Verbindungsrohr (1) umfasst, das an einem ersten Ende (9) von dem ersten Modul (7) umgeben ist und an einem zweiten Ende (10) von dem zweiten Modul (8) umgeben ist, so dass durch das Verbindungsrohr (1) eine pneumatische Verbindung zwischen dem ersten Modul (7) und dem zweiten Modul (8) besteht,
das erste und das zweite Modul (7, 8) jeweils eine Ausnehmung (15) aufweisen und in der Ausnehmung (15) im ersten Modul (7) das erste Ende (9) des Verbindungsrohrs (1) angeordnet ist und in der Ausnehmung (15) im zweiten Modul (8) das zweite Ende (10) des Verbindungsrohrs (1) angeordnet ist,
am ersten Ende (9) des Verbindungsrohrs (1) ein erster elastischer Dichtwulst (19) ausgebildet ist und am zweiten Ende (10) des Verbindungsrohrs (1) ein zweiter elastischer Dichtwulst (19) ausgebildet ist und der erste elastische Dichtwulst (19) in Kontakt mit dem ersten Modul (7) steht und der zweite elastische Dichtwulst (19) in Kontakt mit dem zweiten Modul (8) steht, so dass sich zwischen dem ersten elastischen Dichtwulst (19) und dem ersten Modul (8) eine erste Dichtfläche (16) ausbildet und sich zwischen dem zweiten elastischen Dichtwulst (19) und dem zweiten Modul (8) eine zweite Dichtfläche (16) ausbildet.

2. Beatmungs- oder Anästhesiegerät nach Anspruch 1, wobei das Verbindungsrohr (1) ein gesondertes Bauteil ist.

3. Beatmungs- oder Anästhesiegerät nach Anspruch 1 oder 2, wobei das Verbindungsrohr (1) kein biegbarer Schlauch ist.

4. Beatmungs- oder Anästhesiegerät nach einem der Ansprüche 1 bis 3, wobei das Verbindungsrohr (1) kraft- oder formschlüssig mit dem ersten Modul (7) verbunden ist.

5. Beatmungs- oder Anästhesiegerät nach einem der Ansprüche 1 bis 4, wobei das Verbindungsrohr (1) kraft- oder formschlüssig mit dem zweiten Modul (8) verbunden ist.

6. Beatmungs- oder Anästhesiegerät nach einem der vorhergehenden Ansprüche, wobei das Verbindungsrohr (1) innenseitig ein korrosionsbeständiges Metall und außenseitig ein elastisches Außenmaterial umfasst.

7. Beatmungs- oder Anästhesiegerät nach Anspruch 6, wobei das korrosionsbeständige Metall Edelstahl ist.

8. Beatmungs- oder Anästhesiegerät nach einem der Ansprüche 1 bis 5, wobei das Verbindungsrohr (1) innenseitig einen Kunststoff und außenseitig ein elastisches Außenmaterial umfasst und wobei der Kunststoff ein Polyetheretherketon ist.

9. Beatmungs- oder Anästhesiegerät nach einem der Ansprüche 6 bis 8, wobei das elastische Außenmaterial ein Fluorkautschuk, ein Perfluorkautschuk oder ein Ethylen-Propylen-Kautschuk ist.

10. Beatmungs- oder Anästhesiegerät nach einem der Ansprüche 6 bis 9, wobei der Dichtwulst (19) unter Verwendung des Außenmaterials ausgebildet ist.

11. Beatmungs- oder Anästhesiegerät nach einem der vorhergehenden Ansprüche, wobei das Verbindungsrohr (1) einen kreisförmigen Querschnitt aufweist.

12. Beatmungs- oder Anästhesiegerät nach einem der vorhergehenden Ansprüche, wobei die Querschnittform der Ausnehmung (15) im ersten Modul (7) und/oder im zweiten Modul (8) jeweils der äußeren Querschnittform des Verbindungsrohrs (1) am ersten und/oder zweiten Ende des Verbindungsrohrs (1) entspricht.

13. Beatmungs- oder Anästhesiegerät nach einem der vorhergehenden Ansprüche, wobei die Querschnittform der Ausnehmung (15) im ersten Modul (7) der äußeren Querschnittform des Dichtwulsts (19) entspricht.

14. Beatmungs- oder Anästhesiegerät nach einem der vorhergehenden Ansprüche, wobei das erste Ende (9) und/oder das zweite Ende (10) des Verbindungsrohrs (1) und sein Dichtwulst (19) in einem nicht in die Ausnehmung (15) in den Modulen (6, 7, 8) eingeführten Zustand geringfügig größer ausgebildet ist als die Ausnehmung (15) in den Modulen (6, 7, 8), so dass das erste Ende (9) des Verbindungsrohrs (1) in der Ausnehmung (15) in den Modulen (6, 7, 8) unter einer Vorspannung angeordnet ist.

15. Beatmungs- oder Anästhesiegerät nach einem der vorhergehenden Ansprüche, einschließlich wenigstens eines aus der aus einem Gebläse, einem Atemsystem, einem Mischer und einem Narkosemittelverdunster bestehenden Gruppe ausgewählten Moduls.

## Revendications

1. Respirateur ou machine d'anesthésie (4, 5) qui comprend au moins deux modules (6, 7, 8), au moins un moyen de raccordement pneumatique raccordant les deux modules (6, 7, 8) sur le plan pneumatique, et dans lequel ou laquelle :
le ou les moyens de raccordement pneumatique comprennent un tube de raccordement (1) qui est entouré par le premier module (7) à une première extrémité (9) et par le deuxième module (8) à une seconde extrémité (10), un raccordement pneumatique étant ainsi établi entre le premier module (7) et le deuxième module (8) par le biais du tube de raccordement (1) ;
les premier et deuxième modules (7, 8) comportent chacun un évidement (15), et la première extrémité (9) du tube de raccordement (1) est disposée dans l'évidement (15) dans le premier module (7) et la seconde extrémité (10) du tube de raccordement (1) est disposée dans l'évidement (15) dans le deuxième module (8) ;
un premier bourrelet d'étanchéité (19) élastique est formé au niveau de la première extrémité (9) du tube de raccordement (1) et un second bourrelet d'étanchéité (19) élastique est formé au niveau de la seconde extrémité (10) du tube de raccordement (1), et le premier bourrelet d'étanchéité (19) élastique se trouve en contact avec le premier module (7) et le second bourrelet d'étanchéité (19) élastique se trouve en contact avec le deuxième module (8), une première région d'étanchéité (16) étant ainsi formée entre le premier bourrelet d'étanchéité (19) élastique et
le premier module (8) et une seconde région d'étanchéité (16) étant ainsi formée entre le second bourrelet d'étanchéité (19) élastique et le deuxième module (8).

2. Respirateur ou machine d'anesthésie selon la revendication 1, dans lequel ou laquelle le tube de raccordement (1) est un composant séparé.

3. Respirateur ou machine d'anesthésie selon la revendication 1 ou 2, dans lequel ou laquelle le tube de raccordement (1) n'est pas un tuyau souple.

4. Respirateur ou machine d'anesthésie selon l'une quelconque des revendications 1 à 3, dans lequel ou laquelle le tube de raccordement (1) est raccordé au premier module (7) par le biais d'un accouplement par friction ou par complémentarité de forme.

5. Respirateur ou machine d'anesthésie selon l'une quelconque des revendications 1 à 4, dans lequel ou laquelle le tube de raccordement (1) est raccordé au deuxième module (8) par le biais d'un accouplement par friction ou par complémentarité de forme.

6. Respirateur ou machine d'anesthésie selon l'une quelconque des revendications précédentes, dans lequel ou laquelle le tube de raccordement (1) comprend un métal résistant à la corrosion à l'intérieur et un matériau extérieur élastique à l'extérieur.

7. Respirateur ou machine d'anesthésie selon la revendication 6, dans lequel ou laquelle le métal résistant à la corrosion est de l'acier inoxydable.

8. Respirateur ou machine d'anesthésie selon l'une quelconque des revendications 1 à 5, dans lequel ou laquelle le tube de raccordement (1) comprend une matière plastique à l'intérieur et un matériau extérieur élastique à l'extérieur, et dans lequel ou laquelle la matière plastique est la poly(étheréthercétone).

9. Respirateur ou machine d'anesthésie selon l'une quelconque des revendications 6 à 8, dans lequel ou laquelle le matériau extérieur élastique est un caoutchouc fluoré, un caoutchouc perfluoré ou un caoutchouc d'éthylène et de propylène.

10. Respirateur ou machine d'anesthésie selon l'une quelconque des revendications 6 à 9, et dans lequel ou laquelle le bourrelet d'étanchéité (19) est formé à l'aide du matériau extérieur.

11. Respirateur ou machine d'anesthésie selon l'une quelconque des revendications précédentes, dans lequel ou laquelle le tube de raccordement (1) présente une section transversale circulaire.

12. Respirateur ou machine d'anesthésie selon l'une quelconque des revendications précédentes, dans lequel ou laquelle la forme en section transversale de l'évidement (15) dans le premier module (7) et/ou le deuxième module (8) correspond à la forme en section transversale extérieure du tube de raccordement (1) au niveau respectivement de la première et/ou de la seconde extrémité du tube de raccordement (1).

13. Respirateur ou machine d'anesthésie selon l'une quelconque des revendications précédentes, dans lequel ou laquelle la forme en section transversale de l'évidement (15) dans le premier module (7) correspond à la forme en section transversale extérieure du bourrelet d'étanchéité (19) .

14. Respirateur ou machine d'anesthésie selon l'une quelconque des revendications précédentes, dans lequel ou laquelle, dans un état dans lequel elle n'est pas insérée dans l'évidement (15) dans les modules (6, 7, 8), la première extrémité (9) et/ou la seconde extrémité (10) du tube de raccordement (1), et son bourrelet d'étanchéité (19), est formée de façon à être légèrement plus large que l'évidement (15) dans les modules (6, 7, 8), de telle sorte que la première extrémité (9) du tube de raccordement (1) soit disposée dans l'évidement (15) dans les modules (6, 7, 8) sous une pré-sollicitation.

15. Respirateur ou machine d'anesthésie selon l'une quelconque des revendications précédentes comprenant au moins un module sélectionné dans le groupe constitué d'une turbine, d'un système respiratoire, d'un mélangeur et d'un vaporisateur d'anesthésie.
